# EUROPEAN PATENT SPECIFICATION

(11) **EP 4 507 637 B1**
(45) Date of publication and mention of the grant of the patent: **13.08.2025**
(21) Application number: 23704839.2
(22) Date of filing: 27.01.2023
(51) Int. Cl.: A61F 7/00, A61G 11/00

(54) **HUMAN BODY THERMAL SUPPORT DEVICE**
THERMISCHE UNTERSTÜTZUNGSVORRICHTUNG FÜR DEN MENSCHLICHEN KÖRPER
DISPOSITIF DE SUPPORT THERMIQUE DU CORPS HUMAIN

(30) Priority: 12.04.2022 LV 220029
(43) Date of publication of application: 19.02.2025
(73) Proprietor: Armgate, Sia, Marupe 2167 (LV)
(72) Inventor: VARDANJANS, Reviks, 2010 Jurmala (LV)
(74) Representative: Kromanis, Artis
(86) International application number: PCT/LV2023/050003
(87) International publication number: WO 2023/200322

(56) References cited:
- EP-A1- 0 447 958
- DE-A1- 19 526 103
- GB-A- 2 563 013

## Description

### Field of the invention

The present invention relates to a human body thermal support device.

### Background of the invention

The prior art discloses various designs for human body thermal support. The European patent publication No. EP1520572 discloses a human body thermal support device comprising a frame and an upwardly-facing human body-support surface carried by the frame. The human body-support surface has a perimeter. An air curtain generator is mounted to the frame and provides a first and a second curtains of air. Each of the first and second curtains of air originates adjacent to the perimeter of the surface, and the first and second curtains of air converge at the convergence point above the patient-support surface. The first and second curtains of air cooperate with the patient-support surface to define a patient's space.

Other human body thermal support devices are a human body thermal support gown as disclosed in the US patent publication No. US2012305541 or a human body thermal support drape as disclosed in the US patent publication No. US2010263678. Mentioned devices provide heating only to the regions that are covered by the device but cannot provide a uniformly-warmed or temperature-controlled environment. Besides, these devices work in contact with the human body and may restrict the movements of the patient, and also cause additional difficulties to the caregivers and relatives. And, as these devices are not transparent, they do not allow visual monitoring of the patient, for example, the skin colour changes, possible disconnection of medical supply cannules, etc.

The human thermal support is also very important for new-borns. The prior art discloses various incubators that have some thermal support structures. German patent application publication No. DE19526103 discloses typical incubator comprising two walled enclosure that serves for providing a warmed air to an incubator compartment. The fed warmed air through a two walled enclosure also provides some sort of warming, but is limited to its temperature as it is also used for warming the incubator compartment. Similar designs are disclosed in the European patent application publication No. EP0447958 and in UK patent application publication No. GB2563013.

The aim of the invention is to design a human body thermal support device that may provide warming with and/or without a contact with the human body, as well as may be adapted to various patient warming necessities, for example, infant cot. It may also consist of several parts that may be used together or separately.

### Summary of the invention

The aim is reached by a design of a human body thermal support device comprising a base, a triple-walled wall mounted on the base. The triple-walled wall comprises three walls - an outer wall, an inner wall, and an intermediate wall, forming a first space as an inner side space and a second space as an outer side space. The inner wall is facing the human body when the device is in use. The outer wall is facing the outer environment. The intermediate wall is arranged between the inner wall and the outer wall. The inner wall and the outer wall are spaced apart from the intermediate wall so that between the inner and the intermediate wall the first space is formed, and between the outer wall and the intermediate wall the second space is formed. The first space and the second space are enclosed or separated from the outer environment by the inner wall, the outer wall, the base that encloses both spaces from the bottom, and the upper wall that encloses both spaces from above. The inner side space and the outer side space are fluidly connected to each other through an opening or openings so that a gas flow can flow from the inner side space to the outer side space. The inner wall and the outer wall enclose the spaces so that said spaces are completely sealed from an external environment. Hence, the closed gas flow circulation is provided withing the spaces allowing to control the temperature of the gas flow in more controllable manner as it would be not the case with open circulation. In addition, the closed gas flow circulation requires less energy to maintain the necessary or set-up temperature.

The device comprises a gas flow preparation unit provided at the base of the device. The gas flow preparation unit provides a warmed gas flow. The gas preparation unit is fluidly connected to the triple-walled wall by means of conduits or at least two conduits - an inlet conduit and an outlet conduit. The outlet conduit is fluidly connected to the inner side space, and the inlet conduit is fluidly connected to the outer side space forming a continuous closed or semi-closed flow path from the gas flow preparation unit to the inner side space, through the opening to the outer side space and back to the gas flow preparation unit, as a result of which effective heating of the triple-walled wall is provided. The gas flow preparation unit is configured to warmup and direct said warmed gas flow to and from the triple-walled wall. One of the conduits is fluidly connected to one of the spaces, and another conduit is fluidly connected to another space forming a continuous flow path from the gas flow preparation unit to the first space, through the opening/-s to the second space and back to the gas flow preparation unit, as a result of which effective heating of the triple-walled wall is provided.

From the gas flow preparation unit the gas flow gets into the inner side space through the gas distributing opening/-s. The opening/-s is/are located in the inlet section of the first space so that the gas flow distributes evenly along the first side as the inner side space. The design of the gas openings may be of different forms to direct the gas flow depending on the functions of the device.

The opening/-s is/are located on the intermediate wall and is/are designed to fluidly connect the first space and the second space of the triple-walled wall so that the gas flow travels from the first space as the inner side space into the second space as the outer side space and evenly returns to the inlet section of the gas flow preparation unit. The inlet and outlet sections may be positioned vertically, horizontally or under any other angle.

The gas is selected from the group comprising air, xenon, oxygen, nitrogen, carbon dioxide, helium, or a mixture thereof.

When in use, the human body thermal support device is positioned so that the inner wall of the triple walled wall is facing the human body. The human body is warmed up by the infrared radiation from the inner wall when the warmed gas flow is turned on. The human body thermal support device also reduces the radiation heat losses from the human body to the wall, windows and different objects located behind the device. It also functions as a draught shield. It can be transparent; the human body may be observed through it, and if necessary, it may have a fabric or a special film made from a non-transparent material in order to provide privacy. The device is designed so that it does not have a heating surface with a temperature that may cause burns when touched or in close proximity.

The heated gas prepared in the gas flow preparation unit first enters the first space of the triple-walled wall so that the gas flow with a set temperature reaches the first space facing the patient in the first place. The second space is mainly filled by the gas that outflows from the first space of the triple-walled wall, due to this the gas within the second space has a lower temperature than the gas within the first space as the inner space heat is lost due to radiation. The second space serves as the insulation between the inner environment or a patient side to be warmed and the surrounding environment located opposite from the patient side, thus the gas allows to maintain the necessary temperature in the first space and at the inner wall, increasing the overall effectiveness of the human body thermal support device.

The gas flow preparation unit comprises a heating means. The heating means may be selected from the group consisting of: a gas-conditioned system, an infrared heater, a water/oil-heated radiator, a coiled heater, an open-coil gas heater, a round open-coil gas heater, a convection heater, a straight and formed tubular heater, a quartz tube gas heater, a capacitor-type heater, a thermoelectric heat pump. The gas flow is provided by a fan driven by electricity.

The device features a controller, electronical or mechanical, or a combination of both. The controller controls the fan by means of, for example, a pulse width modulation control signal or an analog control signal, or a digital control protocol. The controller controls the heater by means of a control algorithm, for instance, the Proportional Integral Derivative control algorithm. The controller monitors the real-time parameters of the device, including, but not limited to: the temperatures at different points of the thermal support device, the fan speed, the existence of AC power. The controller evaluates the real-time parameters for compliance with safety algorithms and reacts accordingly. The controller features a display and buttons as human interface devices. The buttons enable operator input, including, but not limited to: changing operation modes, temperature setpoints, fan speed setpoints. The display displays actual operational data of the device, including, but not limited to: real time parameters, setpoints and operation modes, and historic data saved in the memory of the controller. The controller features a buzzer for providing a sound alarm in case of malfunction and/or incompliance of parameters with safety algorithms. The controller features a visual alarm device. Both the visual alarm device and the buzzer may be connected remotely, including, but not limited to by means of LAN (Local Area Network). The controller may have a remote monitoring and control function by means of a wired or wireless connection.

The walls are preferably made of a transparent material. The wall may be made of glass, polymers or combinations thereof. For safety the wall may be laminated so that in the event of breaking it is held in place by said lamination. In addition, the wall may be covered with a metallized or electroconductive layer.

The space between the walls may be filled with or contain a desiccant to remove moisture. The desiccant is arranged within the first space and/or the second space so that it does not obstruct the gas flow within the same space.

One end of the triple wall can also be stationary, i.e., fixed to the wall. Various shelves, lamps, a monitoring stand, a waste bin and medical devices, e.g., a patient monitor, a camera, infusion and suction pumps, etc., can be attached to the base of the stand.

### Brief description of the drawings

The drawings illustrate generally, by way of example, but not by way of limitation, various embodiments of the invention.
Fig. 1 is a perspective view of the human body thermal support device with a close-up view of the gas flow preparation unit (6). The triple-walled wall (2) is removed in order to show the inlet and the outlet from the gas flow preparation unit (6) into the triple-walled wall (2).
Fig. 2 is a front view of two human body thermal support devices.
Fig. 3 is a side view of the human body thermal support devices as seen in Fig. 2.
Fig. 4 is a cross-section view of the triple-walled wall (2) as seen in A-A of Fig. 2
Fig. 5 is a cross-section view of the triple-walled wall (2) as seen in B-B of Fig. 3.
Fig. 6 is a cross-section view of the triple-walled wall (2) as seen in C-C of Fig. 2.

### Detailed description of the embodiments

The preferred embodiments of the invention are now described with reference to the figures to illustrate the objectives, the advantages, and efficiency of the present invention.

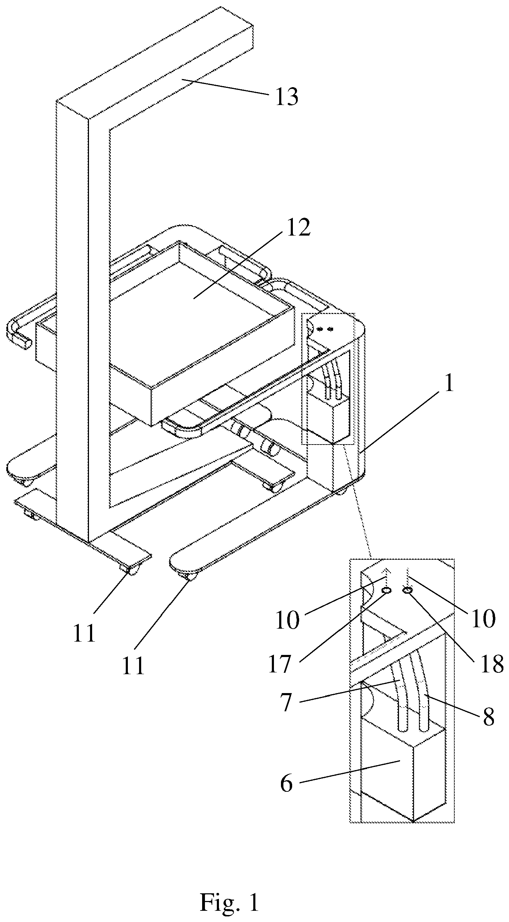

Fig. 1 illustrates the human body thermal support device with a close-up view of the gas flow preparation unit (6). The triple-walled wall (2) is removed in order to show the outlet (17) and the inlet (18) from the gas flow preparation unit (6) into the triple-walled wall (2). Figs. 1 to 3 illustrate the embodiment of the invention where two patient-warming devices with L-type configuration triple-walled wall are used. Both human body thermal support devices are arranged around the patient's bed (12), which in this example is a bed (12) for an infant. The bed (12) is supplemented by a radiant warmer (13). But the radiant warmer (13) does not protect the infant from the body heat loss through radiation to nearby walls or windows. The added human body thermal support device considerably improves the heating capabilities as the temperature of the surfaces of the walls participating in the radiation exchange between the human body and the walls is higher than the human body temperature. In addition, these triple-walled walls minimize draught over the infant and thus reduce the convectional heat losses. The necessary or set-up comfort environment can be maintained within the area of the patient's bed (12). Moreover, the base (1) comprises wheels (11) so that the human body thermal support device is mobile. It allows various configurations or arrangements of the human body thermal support device. For example, the human body thermal support device may be removed during medical or other manipulations with the patient in the bed (12) and then moved back after completing the necessary manipulations.

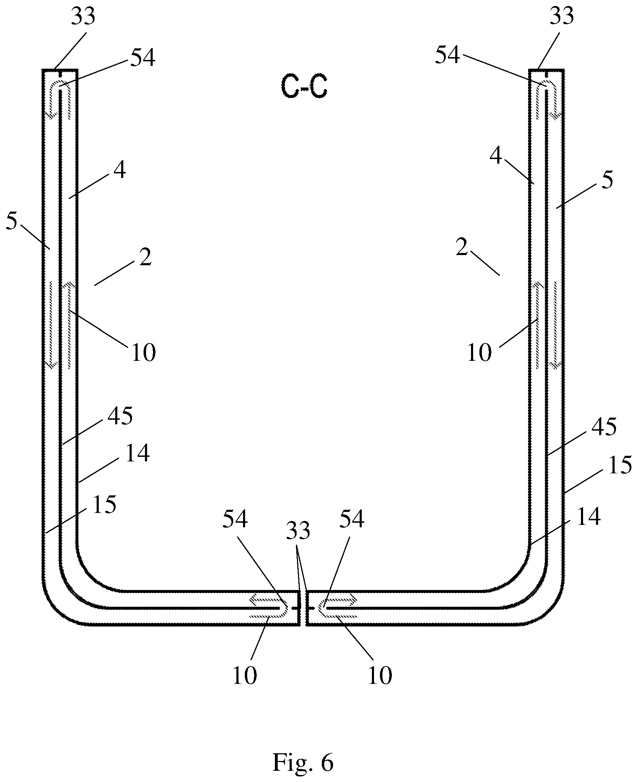

The human body thermal support device comprises a gas flow preparation unit (6) provided at the base (1) as seen in Figs. 1 to 3. The gas flow preparation unit (6) is fluidly connected to the triple-walled wall (2) by means of conduits (7; 8) and further - by means of an outlet conduit (7) and an inlet conduit (8). The gas flow preparation unit (6) is configured to warm-up and direct said warmed gas flow (10) through the outlet conduit (7) and its outlet (17) into the first space (4). The inlet conduit (8) with its inlet (18) is fluidly connected to the second space (5) in order to receive the gas flow (10) that already passed through the first space (4) and the second space (5). The gas preparation unit (6) comprises a heating means for heating the gas, as well as a ventilator for generating a warmed gas flow (10). The arrangement of the first space (4) and the second space (5) is seen in more detail in Fig. 6.

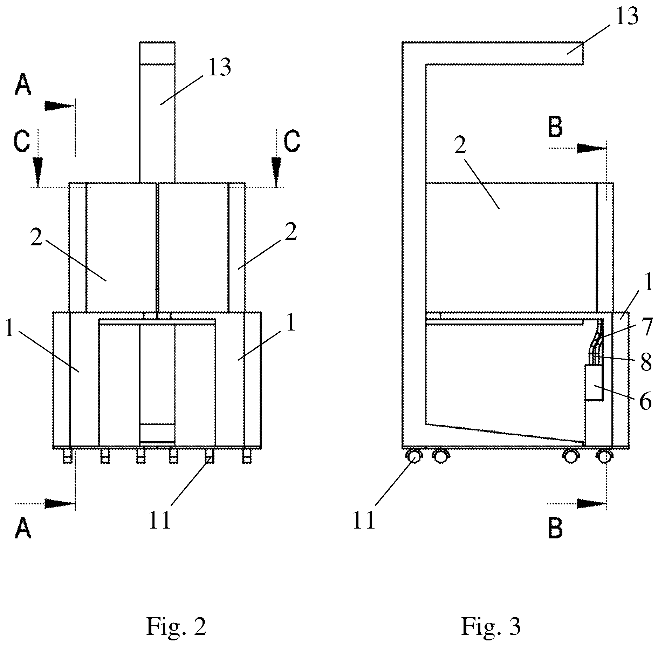

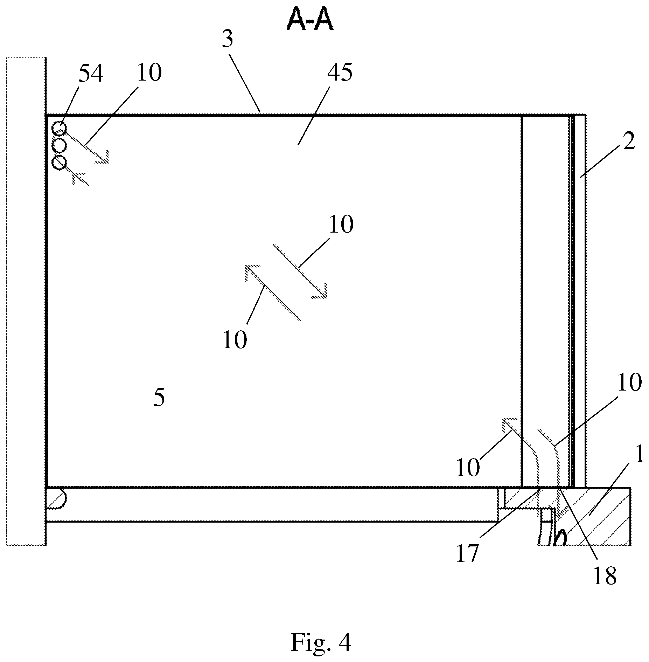

Fig. 4 is a cross-section view of the triple-walled wall (2) as seen in A-A of Fig. 2. The cross section is made in the second space (5) of the triple-walled wall (2). The intermediate wall (45) of the triple-walled wall (2) with openings (54) is seen in Fig. 4. The gas flow (10) flows out from the outlet (17) into the first space (4), then it reaches the three openings (54) and flows into the second space (5) from where the gas flow (10) goes into the inlet (18). Three openings (54) are arranged in the upper section and on the side of the intermediate wall (45) of the triple-walled wall (2). The second space (5) is enclosed from all sides by the base (1), the outer wall (15), the intermediate wall (45), and the upper wall (3) so that the gas does not escape it and is transferred only through the openings (54) to the inlet (18).

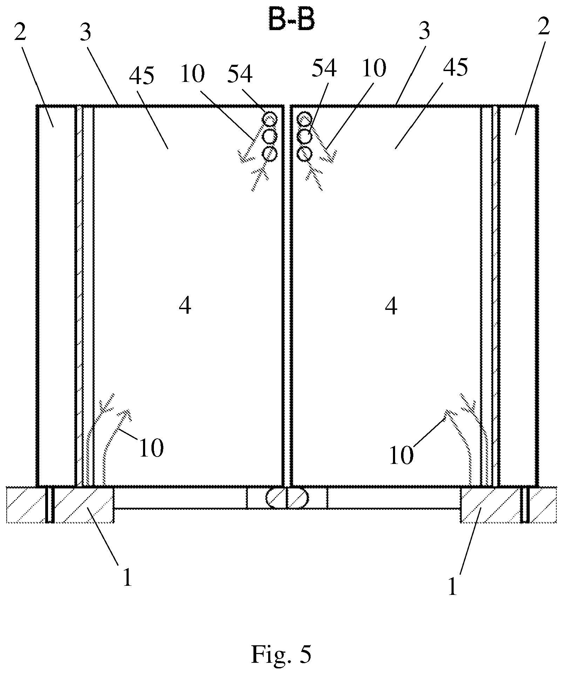

Fig. 5 is a cross-section view of the triple-walled wall (2) as seen in B-B of Fig. 3. The cross section is made in the first space (4) of the triple-walled wall (2). The intermediate wall (45) of the triple-walled wall (2) with openings (45) is seen as well in Fig. 5. Three openings (54) are arranged in the upper section and on the side of the intermediate wall (45) of the triple-walled wall (2). The first space (4) is enclosed from all sides by the base (1), the inner wall (14), the intermediate wall (45), and the upper wall (3) so that the gas does not escape it and is transferred only through the outlet (17) and the openings (54).

The gas flow preparation unit (6), the outlet conduit (7) with its outlet (17), the first space (4), the second space (5), and the inlet conduit (8) with its inlet (18) are interconnected to one another so that a closed gas flow (10) circulation system is created.

Fig. 6 is a cross-section view of the triple-walled wall (2) as seen in C-C of Fig. 2. This cross section from above illustrates all three walls (14; 45; 15) of the triple-walled wall (2). The intermediate wall (45) comprises openings (54) on each side of the triple-walled wall (2). The triple-walled wall (2) has an L-type configuration allowing to enclose the warmable space, for example, the patient's bed, at least from two sides. When two devices are arranged to each other, the patient's bed can be warmed already from three sides.

Figs. 5 and 6 illustrate a triple walled wall (2) comprising the inner wall (14), the outer wall (15), and the intermediate wall (45) therebetween. The inner wall (14) and the outer wall (15) enclose the spaces (4, 5) so that said spaces (4, 5) are completely sealed from an external environment. Hence, the closed gas flow (10) circulation is provided allowing to control the temperature of the gas flow (10) in more controllable manner as it would be not the case with open circulation. In addition, the closed gas flow (10) circulation requires less energy to maintain the necessary temperature.

### List of references

1 - a base;
2 - a triple-walled wall;
3 - an upper wall;
4 - a first space;
5 - a second space;
6 - a gas flow preparation unit
7 - an outlet conduit;
8 - an inlet conduit;
10 - a gas flow;
11 - a wheel;
12 - a bed;
13 - a radiant warmer;
14 - an inner wall;
15 - an outer wall;
17 - an outlet;
18 - an inlet;
33 - an end wall;
45 - an intermediate wall; and
54 - an opening.

## Claims

1. A human body thermal support device comprising:
a base (1),
a triple-walled wall (2) mounted on the base (1), wherein the triple-walled wall (2) comprises an inner wall (14), an outer wall (15), an intermediate wall (45) therebetween, and an upper wall (3) and wherein the inner wall (14) and the outer wall (15) are spaced apart from the intermediate wall (45) so that between the inner wall (14) and the intermediate wall (45) a first space (4) is formed, and between the outer wall (15) and the intermediate wall (45) a second space (5) is formed, and wherein the first space (4) and the second space (5) are fluidly connected to each other through an opening (54) in the intermediate wall (45) so that a gas flow (10) can flow from the first space (4) to the second space (5),
a gas flow preparation unit (6) provided at the base (1), wherein the gas flow preparation unit (6) is fluidly connected to the first space (4) and the second space (5) of the triple-walled wall (2) by means of conduits (7;8), and wherein the gas flow preparation unit (6) is configured to warm-up and direct said warmed gas flow (10) to the first space (4) and extract from the second space (5) of the triple-walled wall (2);
wherein one of the conduits (7;8) is fluidly connected to one of the spaces (4;5), and another conduit (7;8) is fluidly connected to another space (4;5) forming a continuous flow path from the gas flow preparation unit (6) to the first space (4), through the opening (54) to the second space (5) and back to the gas flow preparation unit (6), as a result of which effective heating of the triple-walled wall (2) is provided,
wherein the first space (4) is enclosed from all side by the base (1), the inner wall (14), the intermediate wall (45) and the upper wall (3) so that the gas does not escape it and is transferred only through opening in the intermediate wall to one of the conduits, and
the second space (5) is enclosed from all sides by the base (1), the outer wall (15), the intermediate wall (45) and the upper wall (3) so that the gas does not escape it and is transferred only through opening in the intermediate wall to one of the conduits.

2. The device according to claim 1, **characterized in that** the opening (54) is located in the upper section and/or in the side section of the intermediate wall (45) of the triple-walled wall (2).

3. The device according to claim 1 or 2, **characterized in that** baffles are arranged in the first space (4) and/or the second space (5) of the device for directing the gas flow vertically, horizontally or under any angle.

4. The device according to any of the preceding claims 1 to 3, **characterized in that** the inner wall (14), the outer wall (15) and the intermediate wall (45) is/are made of a transparent material.

5. The device according to any of the preceding claims 1 to 4, **characterized in that** the inner wall (14), the outer wall (15) and the intermediate wall (45) is/are covered with a metallized or electroconductive layer.

6. The device according to any of the preceding claims 1 to 5, **characterized in that** the first space (4) of the triple-walled wall (2) is oriented closer to the patient or the area to be warmed than the second space (5) of the triple-walled wall (2).

7. The device according to any of the preceding claims 1 to 6, **characterized in that** it further comprises a bed (12), wherein the bed (12) is partly enclosed by the triple-walled wall (2) so that the first space (4) of the triple-walled wall (2) is oriented closer to the bed (12) than the second space (5).

## Patentansprüche

1. Thermische Stützvorrichtung für den menschlichen Körper, umfassend:
eine Basis (1),
eine dreiwandige Wand (2), die an der Basis (1) angebracht ist, wobei die dreiwandige Wand (2) eine Innenwand (14), eine Außenwand (15), eine Zwischenwand (45) dazwischen und eine obere Wand (3) umfasst und wobei die Innenwand (14) und die Außenwand (15) von der Zwischenwand (45) beabstandet sind, so dass zwischen der Innenwand (14) und der Zwischenwand (45) ein erster Raum (4) gebildet wird, und zwischen der Außenwand (15) und der Zwischenwand (45) ein zweiter Raum (5) gebildet wird, und wobei der erste Raum (4) und der zweite Raum (5) durch eine Öffnung (54) in der Zwischenwand (45) strömungstechnisch miteinander verbunden sind, so dass ein Gasstrom (10) von dem ersten Raum (4) zu dem zweiten Raum (5) fließen kann,
eine Gasströmungsvorbereitungseinheit (6), die an der Basis (1) vorgesehen ist, wobei die Gasströmungsvorbereitungseinheit (6) mit dem ersten Raum (4) und dem zweiten Raum (5) der dreiwandigen Wand (2) mittels Leitungen (7; 8) in Fluidverbindung steht, und wobei die Gasströmungsvorbereitungseinheit (6) so konfiguriert ist, dass sie den erwärmten Gasstrom (10) erwärmt und zu dem ersten Raum (4) leitet und aus dem zweiten Raum (5) der dreiwandigen Wand (2) abzieht; wobei eine der Leitungen (7; 8) mit einem der Räume (4; 5) strömungstechnisch verbunden ist und eine andere Leitung (7; 8) mit einem anderen Raum (4; 5) strömungstechnisch verbunden ist, wodurch ein kontinuierlicher Strömungsweg von der Gasströmungsvorbereitungseinheit (6) zum ersten Raum (4), durch die Öffnung (54) zum zweiten Raum (5) und zurück zur Gasströmungsvorbereitungseinheit (6) gebildet wird, wodurch eine wirksame Erwärmung der dreifachwandigen Wand (2) bewirkt wird,
wobei der erste Raum (4) von allen Seiten durch den Boden (1), die Innenwand (14), die Zwischenwand (45) und die obere Wand (3) umschlossen ist, so dass das Gas nicht aus ihm entweicht und nur durch eine Öffnung in der Zwischenwand zu einer der Leitungen geleitet wird, und
der zweite Raum (5) von allen Seiten von dem Boden (1), der Außenwand (15), der Zwischenwand (45) und der oberen Wand (3) umschlossen ist, so dass das Gas nicht entweicht und nur durch eine Öffnung in der Zwischenwand in eine der Leitungen geleitet wird.

2. Vorrichtung nach Anspruch 1, **dadurch gekennzeichnet, dass** die Öffnung (54) im oberen Bereich und/oder im Seitenbereich der Zwischenwand (45) der dreiwandigen Wand (2) angeordnet ist.

3. Vorrichtung nach Anspruch 1 oder 2, **dadurch gekennzeichnet, dass** in dem ersten Raum (4) und/oder dem zweiten Raum (5) der Vorrichtung Leitbleche angeordnet sind, um den Gasstrom vertikal, horizontal oder unter einem beliebigen Winkel zu lenken.

4. Vorrichtung nach einem der vorhergehenden Ansprüche 1 bis 3, **dadurch gekennzeichnet, dass** die Innenwand (14), die Außenwand (15) und die Zwischenwand (45) aus einem transparenten Material besteht/bestehen.

5. Vorrichtung nach einem der vorhergehenden Ansprüche 1 bis 4, **dadurch gekennzeichnet, dass** die Innenwand (14), die Außenwand (15) und die Zwischenwand (45) mit einer metallisierten oder elektrisch leitenden Schicht bedeckt ist/sind.

6. Vorrichtung nach einem der vorhergehenden Ansprüche 1 bis 5, **dadurch gekennzeichnet, dass** der erste Raum (4) der dreiwandigen Wand (2) näher zum Patienten oder dem zu wärmendes Bereich ausgerichtet ist als der zweite Raum (5) der dreiwandigen Wand (2).

7. Vorrichtung nach einem der vorhergehenden Ansprüche 1 bis 6, **dadurch gekennzeichnet, dass** sie ferner ein Bett (12) umfasst, wobei das Bett (12) teilweise von der dreifachwandigen Wand (2) umschlossen ist, so dass der erste Raum (4) der dreifachwandigen Wand (2) näher zum Bett (12) hin ausgerichtet ist als der zweite Raum (5).

## Revendications

1. Dispositif de soutien thermique du corps humain comprenant
une base (1),
une paroi à triple paroi (2) montée sur la base (1), dans laquelle la paroi à triple paroi (2) comprend une paroi intérieure (14), une paroi extérieure (15), une paroi intermédiaire (45) entre les deux et une paroi supérieure (3), et dans laquelle la paroi intérieure (14) et la paroi extérieure (15) sont espacées de la paroi intermédiaire (45) de sorte qu'entre la paroi intérieure (14) et la paroi intermédiaire (45), un premier espace (4) est formé, et entre la paroi extérieure (15) et la paroi intermédiaire (45) un second espace (5) est formé, et dans lequel le premier espace (4) et le second espace (5) sont reliés de manière fluide l'un à l'autre par une ouverture (54) dans la paroi intermédiaire (45) de sorte qu'un flux de gaz (10) puisse circuler du premier espace (4) au second espace (5),
une unité de préparation du flux de gaz (6) prévue à la base (1), dans laquelle l'unité de préparation du flux de gaz (6) est reliée fluidiquement au premier espace (4) et au second espace (5) de la paroi à triple paroi (2) au moyen de conduits (7;8), et dans laquelle l'unité de préparation du flux de gaz (6) est configurée pour réchauffer et diriger ledit flux de gaz réchauffé (10) vers le premier espace (4) et l'extraire du second espace (5) de la paroi à triple paroi (2) ;
dans lequel l'un des conduits (7;8) est relié de manière fluide à l'un des espaces (4;5), et un autre conduit (7;8) est relié de manière fluide à un autre espace (4;5) formant un chemin d'écoulement continu de l'unité de préparation du flux de gaz (6) au premier espace (4), à travers l'ouverture (54) au second espace (5) et de nouveau à l'unité de préparation du flux de gaz (6), ce qui permet de chauffer efficacement la paroi triple (2),
le premier espace (4) est fermé de tous les côtés par la base (1), la paroi intérieure (14), la paroi intermédiaire (45) et la paroi supérieure (3), de sorte que le gaz ne s'en échappe pas et n'est transféré qu'à travers une ouverture dans la paroi intermédiaire vers l'un des conduits, et que
le second espace (5) est entouré de tous côtés par la base (1), la paroi extérieure (15), la paroi intermédiaire (45) et la paroi supérieure (3), de sorte que le gaz ne s'en échappe pas et n'est transféré à l'un des conduits que par une ouverture pratiquée dans la paroi intermédiaire.

2. Le dispositif selon la revendication 1, **caractérisé en ce que** l'ouverture (54) est située dans la partie supérieure et/ou dans la partie latérale de la paroi intermédiaire (45) de la paroi triple (2).

3. Le dispositif selon la revendication 1 ou 2, **caractérisé en ce que** des chicanes sont disposées dans le premier espace (4) et/ou le second espace (5) du dispositif pour diriger le flux de gaz verticalement, horizontalement ou sous n'importe quel angle.

4. Le dispositif selon l'une quelconque des revendications précédentes 1 à 3, **caractérisé en ce que** la paroi intérieure (14), la paroi extérieure (15) et la paroi intermédiaire (45) est/sont constituée(s) d'un matériau transparent.

5. Le dispositif selon l'une quelconque des revendications précédentes 1 à 4, **caractérisé en ce que** la paroi intérieure (14), la paroi extérieure (15) et la paroi intermédiaire (45) est/sont recouverte(s) d'une couche métallisée ou électroconductrice.

6. Le dispositif selon l'une des revendications précédentes 1 à 5, **caractérisé par le fait que** le premier espace (4) de la paroi à triple paroi (2) est orienté plus près du patient ou de la zone à réchauffer que le second espace (5) de la paroi à triple paroi (2).

7. Le dispositif selon l'une des revendications précédentes 1 à 6, **caractérisé en ce qu'**il comprend en outre un lit (12), dans lequel le lit (12) est partiellement entouré par la paroi à triple paroi (2) de sorte que le premier espace (4) de la paroi à triple paroi (2) est orienté plus près du lit (12) que le second espace (5).
